# EUROPÄISCHE PATENTSCHRIFT

(11) **EP 0 515 807 B1**
(45) Veröffentlichungstag und Bekanntmachung des Hinweises auf die Patenterteilung: **07.06.1995**
(21) Anmeldenummer: 92106128.9
(22) Anmeldetag: 09.04.1992
(51) Int. Cl.: A61M 25/02

(54) **Haftverband für intravenöse Katheter**
Adhesive dressing for intravenous catheter
Pansement adhésif pour cathéter intraveineux

(30) Priorität: 27.05.1991 DE 4117282
(43) Veröffentlichungstag der Anmeldung: 02.12.1992
(73) Patentinhaber: Beiersdorf Aktiengesellschaft, 20245 Hamburg (DE)
(72) Erfinder: Lütten, Axel, W-2000 Hamburg 52 (DE)

(56) Entgegenhaltungen:
- EP-A- 0 117 632
- EP-A- 0 168 174
- EP-A- 0 254 696
- EP-A- 0 284 219
- WO-A-90/04429
- GB-A- 2 035 096
- US-A- 3 834 380

## Beschreibung

Die Erfindung betrifft einen Haftverband für intravenöse Katheter oder dgl. aus einem Trägermaterial mit einer Selbstklebeschicht an seiner Unterseite, die mit einer zumindest zweiteiligen, abziehbaren Schutzabdeckung versehen ist, und mit einem im wesentlichen mittigen, von einem Rand des Verbandes ausgehenden, diesen teilweise trennenden Einschnitt. Gegebenenfalls kann der Haftverband auch mit einer saugenden Wundauflage versehen sein.

Haftverbände dieser oder ähnlicher Art sind an sich bekannt, wie beispielsweise aus der DE-C 29 47 427, DE-A 38 36 828, EP-A 254 696, EP-A 284 219 sowie der US-A 3,918,446 und 4,324,237. Sie dienen der Fixierung vor allem von Venenkathetern, die über längere Zeit eingesetzt werden, und sollen zugleich die Einstichstelle steril abdecken sowie gegebenenfalls austretende kleine Mengen an Blut aufsaugen.

Sie bestehen vorzugsweise aus anschmiegsamen, relativ dünnen Klebematerialien, deren Handhabung und einwandfreies Anbringen immer ein gewisses Problem darstellt. Die bekannten Pflaster erfüllen die an sie gestellten Anforderungen nicht in vollem Umfang, insbesondere was beispielsweise die einfache Handhabbarkeit betrifft, wegen der Ausgestaltung ihrer Schutzabdeckung über der Klebeschicht, so daß es Aufgabe der Erfindung war, einen verbesserten Haftverband zu entwickeln, der sich einfach anbringen und außerdem günstig herstellen läßt.

Gelöst wird diese Aufgabe durch einen Haftverband der eingangs genannten Art, der dadurch gekennzeichnet ist, daß die Teilungslinie der Schutzabdeckung parallel zu dem Einschnitt, jedoch versetzt zu diesem in einer Hälfte des Verbandes verläuft.

Ein Positionieren der Trennungslinie um etwa 1/8 bis 1/4, vorteilhafterweise etwa 1/6 aus der Mitte in eine Hälfte des Pflasters hat sich als günstig erwiesen.

Der Einschnitt in den Haftverband erleichtert die Fixierung der üblichen Flügelkanülen, indem die beiden durch den Einschnitt entstandenen Lappen des Pflasters über die seitlichen Flügel gelegt und mit der umgebenden Haut verklebt werden. Dieser Einschnitt kann ein bloßer Schnitt sein, vorzugsweise jedoch ist er als längliche Aussparung, besonders vorteilhaft als U-förmige Aussparung ausgebildet, um Platz für das Zuspritzteil der Kanüle zu schaffen. Die U-förmige Aussparung ist vorzugsweise etwa 5-10 mm breit und reicht bis etwa in die Mitte des Pflasters.

Eine weitere Ausführungsform des Haftverbandes ist dadurch gekennzeichnet, daß er nicht einen von einem Rand des Verbandes ausgehenden Einschnitt oder eine Aussparung aufweist, sondern daß er etwa auf der Mittellinie in einer Hälfte des Pflasters eine vorzugsweise längliche Aussparung, die vor dem Rand endet, besitzt. Die Aussparung wird ebenfalls bei der Anwendung um das Zuspritzteil der Kanüle herumgelegt.

Die Teilungslinie der Schutzabdeckung ist wieder erfindungsgemäß parallel zu der Aussparung, jedoch versetzt zu dieser, in eine Hälfte des Verbandes verlegt.

Die weiteren Darlegungen beziehen sich auf beide oben beschriebenen Ausführungsformen des Haftverbandes.

Um die Schutzabdeckung, die üblicherweise aus einem klebstoffabweisenden Material, wie silikonisiertes Papier besteht, leichter vom Verband abziehen zu können, weisen die beiden Teile jeweils entlang ihrer Trennlinie vorzugsweise Anfaßlaschen auf, die in an sich bekannter Weise beide zurückgefaltet sein können oder - noch besser - von denen eine zurückgefaltet ist und die andere diese teilweise überlappt.

Das Trägermaterial des erfindungsgemäßen Haftverbandes sollte aus einem relativ dünnen, schmiegsamen, luft- und wasserdampfdurchlässigen Material bestehen, wie Z.B. einer entsprechenden Folie, einem Gewebe oder Vlies. Vorzugsweise eignen sich die besonders hautverträglichen, dehnbaren, wasserdampfdurchlässigen dünnen und transparenten Polyurethanfolien aus elastischen, thermoplastischen Polyurethanen wie sie in der DE-C 19 34 710 beschrieben und offenbart sind. Besonders vorteilhaft haben sich aliphatische Polyesterurethane erwiesen. Eine bevorzugte Folie ist etwa 30 - 40 Mikrometer stark, transparent, weist eine Reißdehnung von über 450% und eine Wasserdampfdurchlässigkeit von über 500 g/m² in 24h bei 38°C und 95% rel. Feuchte nach DAB auf.

Daneben lassen sich jedoch auch Filme auf anderer Grundlage, wie z.B. Acrylat-Copolymere oder die anderen bekannten filmbildenden elastischen Polymeren, verwenden.

Bei der Verwendung derartiger Folien hat es sich als günstig erwiesen, den Haftverband auf seiner nichtklebenden Seite mit einem leicht wiederablösbaren Hilfsträger zu versehen. Vorzugsweise wird ein Hilfsträger mit überstehenden Griffleisten gemäß der nicht vorveröffentlichten Patentanmeldung P 40 26 755.5 eingesetzt.

Das Hilfsträgermaterial, das eine stützende Wirkung für die Folie hat und beim Aufbringen des Verbandes auf dieser verbleibt, besteht vorzugsweise aus einer Polyethylenfolie von etwa 80 Mikrometer Dicke, die auf ihrer Seite zur Folie hin leicht gerauht ist und dadurch matt, aber noch durchscheinend wirkt.

Es lassen sich auch andere Folien aus beispielsweise Polypropylen, Polyester, PVC oder geeignetes dünnes, beschichtetes Papier verwenden, sofern sie nur schmiegsam genug sind, um beim Anbringen des Verbandes nicht zu stören.

Die Griffleisten, die, in Längsrichtung des Verbandes gesehen, rechts und links außen auf den Rändern des Hilfsträgers etwa 3 cm breit und davon etwa 2 cm überstehend angebracht sind, bestehen vorzugsweise aus einer LDPE-Folie (low density polyethylene-Folie) von ebenfalls 80 Mikrometer Dicke. Um gut sichtbar zu sein, sind sie beispielsweise blau eingefärbt und wegen der besseren Griffigkeit an der Außenseite matt geprägt.

Anstelle dieser Folie können auch andere Materialien wie HDPE-, Polypropylen-, PVC-, PU- oder Polyesterfolien sowie Vliese, Papier oder Gewebe verwendet werden, sofern sie nur wieder ausreichend flexibel und anschmiegsam sind.

Die Befestigung der Griffleisten an dem Hilfsträgermaterial kann je nach Material und Verarbeitungsmaschinen in verschiedenster Weise erfolgen, vorzugsweise durch Kleben oder Verschweißen. Das Kleben erfolgt dabei beispielsweise in der Weise, daß zwischen das Hilfsträgermaterial und den Griffleistenstreifen im Überlappungsbereich ein Streifen eines doppelseitig klebenden Klebebandes eingebracht wird oder eine Klebemassenbeschichtung aus einer Hotmelt-Masse oder einer Selbstklebemasse aus Lösungsmittel oder Dispersion.

Die Haftung der Folie auf dem Hilfsträger, die nur gering zu sein braucht, wird vorzugsweise dadurch bewirkt, daß die dünne Folie direkt auf dem Hilfsträger erzeugt wird durch Gießen, Rakeln, Extrudieren oder andere bekannte Methoden zur Filmherstellung. Falls notwendig, kann das Hilfsträgermaterial auf der Beschichtungsseite aufgerauht oder einer anderen haftungsfördernden Behandlung unterworfen werden. Auch eine adhäsionsfördernde Beschichtung kann vorteilhaft sein.

Wichtig dabei ist, daß die Haftung des fertigen Verbandes auf der Haut wesentlich stärker ist als die Haftung des Hilfsträgers an der Folie.

Der Haftverband kann als solcher zur Fixierung verwendet werden, es kann jedoch auch zusätzlich etwa mittig in geeigneter Breite eine übliche, saugende Wundauflage angebracht sein zur Aufnahme von evtl. austretendem Blut und zur Abpolsterung und zum Schutz der Einstichstelle.

Als besonders vorteilhaft hat es sich erwiesen, wenn die Wundauflage in der Weise ausgestaltet ist, daß sie sich teilweise oberhalb des Einschnitts bzw. der Aussparung befindet und sich teilweise noch entlang der Seitenränder des Einschnitts bzw. der Aussparung erstreckt. Vorzugsweise erstreckt sich die Wundauflage entlang etwa 2/3 - 3/4 der Länge des Einschnitts bzw. der Aussparung.

Die Selbstklebeschicht besteht üblicherweise aus einer gut hautverträglichen Klebemasse, beispielweise auf Arcylatbasis, die vorzugsweise im Transferverfahren aufgebracht worden ist, und kann zusätzlich eine geeignete entzündungshemmende, antibakterielle Substanz, wie Silber-Sulfadiazin, enthalten.

Die Schutzabdeckung über der Selbstklebeschicht ist zwar vorzugsweise zweiteilig ausgebildet und durch die Verschiebung der Teilungslinie in eine Hälfte des Verbandes asymmetrisch, sie kann jedoch auch bei entsprechendem Bedarf mehrteilig und symmetrisch ausgeführt sein. Beispielsweise in der Form, daß sie in Längsrichtung des Verbandes in 3 Streifen aufgeteilt ist und die beiden Teilungslinien jeweils parallel zu dem Einschnitt bzw. der Aussparung sowie versetzt zu diesen in der linken und rechten Hälfte des Verbandes verlaufen. Anfaßlaschen entlang der zueinanderweisenden Ränder erleichtern wieder das Abziehen.

Die Schutzabdeckung ist bei allen Ausführungsformen vorzugsweise etwas breiter als das Trägermaterial, um dessen Randkanten zu schützen.

Der erfindungsgemäße Haftverband wird in Form einzelner vorgefertigter Abschnitte und evtl. durch Gamma-Strahlen sterilisiert und eingesiegelt in verschiedenen Längen und Breiten angeboten. Beim Gebrauch wird einfach nur die Schutzabdeckung über der Klebeschicht entfernt, das Material beidseitig an den Griffleisten erfaßt und bedarfsmäßig aufgeklebt.

Anschließend wird das leicht wiederablösbare Trägermaterial auf der Rückseite der Folie mit den Griffleisten rückstandsfrei abgezogen.

Auch bei Haftverbänden ohne Hilfsträger und damit ohne überstehende Griffleisten, wobei bei diesen der Träger vorteilhafterweise aus einem Material mit größerer Eigenfestigkeit besteht, z.B. aus Vlies oder Acetatseide, läßt sich durch Abziehen des kleineren Teils der Schutzabdeckung und Aufkleben bzw. Arretieren des Produktes und anschließendes Entfernen des zweiten größeren Teils der Schutzabdeckung eine einfache und sichere Applikation erreichen.

Die erfindungsgemäße Ausführungsform der Schutzabdeckung mit einer Teilungslinie, die in Längsrichtung außerhalb der Mitte des Verbandes verläuft, hat gegenüber den vorbekannten Ausführungsformen, bei denen die Teilungslinie der zwei- oder dreiteiligen Abdeckung quer zum Verband verläuft, den wesentlichen Vorteil, daß die Anfaßlaschen unbeeinträchtigt von dem Einschnitt oder der Aussparung über die ganze Länge ihre Funktion als Anfasser behalten. Sie werden von dem Stanzvorgang für die Aussparung nicht betroffen. Obwohl nur zwei Teile entfernt werden müssen entstehen keine lose hängenden Lappen, die sich in unerwünschter Weise verkleben können, wie beispielsweise bei einer Abdeckung gemäß der DE-PS 29 47 427. Das Abziehen des Trennpapiers und das anschließende Aufkleben des Pflasters ist dadurch problemlos möglich.

Durch die in Längsrichtung des Verbandes verlaufende Teilungslinie der Schutzabdeckung gleicht die Applikation der Handhabung der üblichen Wundpflaster. Es sind also nur die gleichen geübten Handgriffe notwendig.

Diese Art der Schutzabdeckung ist außerdem bei der Herstellung des Verbandes besonders günstig, da in Laufrichtung der Maschinen eingedeckt und ausgestanzt werden kann. Der Materialabfall, der beim Ausstanzen der Aussparung aus dem Träger und gegebenenfalls der Wundauflage anfällt ist gering.

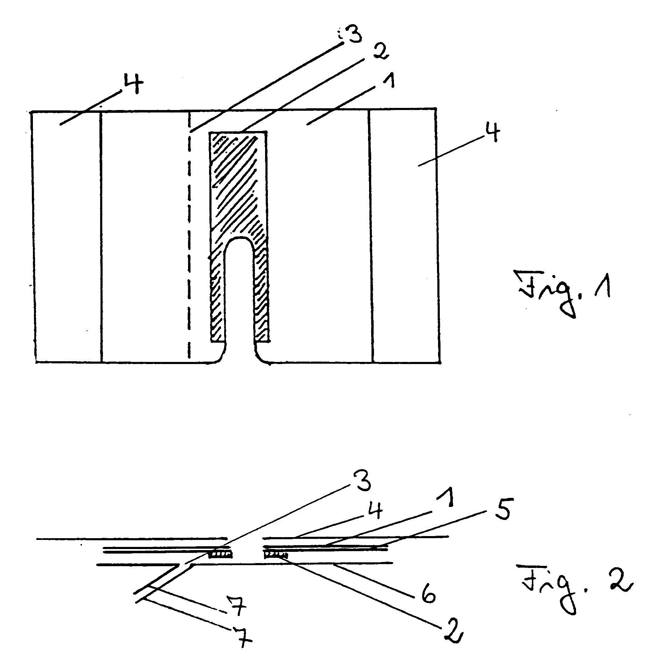

Der erfindungsgemäße Haftverband ist schematisch in den Figuren 1 - 4 beispielsweise dargestellt und zwar in jeweils einer Ausführungsform mit Wundauflage. Die Wundauflagen sind jedoch nicht obligatorisch.

In Figur 1 bedeutet 1 das Trägermaterial, 2 die Wundauflage, 3 die Trennungslinie der Schutzabdeckung, wobei die Anfaßlaschen nicht dargestellt sind, sowie 4 die überstehenden Griffleisten des Hilfsträgers auf der Rückseite des Trägers. Der Haftverband ist maßstabsgetreu 1:1 dargestellt.

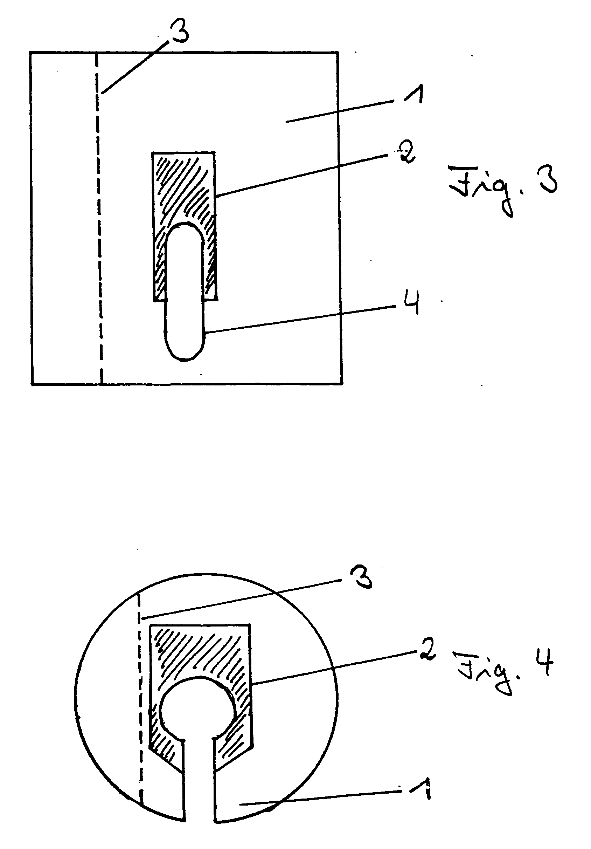

In Figur 2 ist derselbe Haftverband im Querschnitt dargestellt. 1 bedeutet wieder das Trägermaterial, 2 die Wundauflage, 3 die Trennungslinie der Schutzabdeckung, 6 die Schutzabdeckung und 7 die Anfaßlaschen der Schutzabdeckung, 4 den Hilfsträger mit den überstehenden Griffleisten und 5 die Selbstklebeschicht auf der Unterseite des Trägermaterials 1.

In Figur 3 ist eine Ausführungsform des Haftverbandes dargestellt ohne Hilfsträger mit überstehenden Anfaßlaschen und mit einer Aussparung, welche nicht bis zum Rand des Pflasters reicht, sondern innerhalb von diesem verbleibt. 1 bedeutet das Trägermaterial, 2 die Wundauflage, 3 die Trennungslinie der Schutzabdeckung und 4 die Aussparung.

In Figur 4 ist eine weitere, runde Ausführungsform dargestellt mit variierter Form der Aussparung und entsprechender Wundauflage. 1 bedeutet das Trägermaterial, 2 die Wundauflage und 3 die Trennungslinie der Schutzabdeckung.

## Patentansprüche

1. Haftverband für intravenöse Katheter oder dgl. aus einem Trägermaterial (1) mit einer Selbstklebeschicht (5) an seiner Unterseite, die mit einer zumindest zweiteiligen, abziehbaren Schutzabdeckung (6) versehen ist, und einem mittigen, von einem Rand des Verbandes ausgehenden, diesen teilweise trennenden Einschnitt, dadurch gekennzeichnet, daß die Teilungslinie (3) der Schutzabdeckung parallel zu dem Einschnitt, jedoch versetzt zu diesem, in einer Hälfte des Verbandes verläuft.

2. Haftverband gemäß Anspruch 1, dadurch gekennzeichnet, daß der Einschnitt als längliche Aussparung in beliebiger Form ausgebildet ist.

3. Haftverband gemäß Anspruch 1 oder 2, dadurch gekennzeichnet, daß der Einschnitt als U-förmige Aussparung ausgebildet ist.

4. Haftverband für intravenöse Katheter oder dgl. aus einem Trägermaterial (1) mit einer Selbstklebeschicht (5) an seiner Unterseite, die mit einer zumindest zweiteiligen, abziehbaren Schutzabdeckung (6) versehen ist, dadurch gekennzeichnet, daß er etwa auf der Mittellinie in einer Hälfte des Verbandes eine vorzugsweise längliche Aussparung aufweist und daß die Teilungslinie (3) Schutzabdeckung parallel zu der Aussparung, jedoch versetzt zu dieser, in einer Hälfte des Verbandes verläuft.

5. Haftverband gemäß einem der Ansprüche 1 bis 4, dadurch gekennzeichnet, daß die Schutzabdeckungsteile jeweils entlang ihrer Teilungslinie mit Anfaßlaschen (7) versehen sind.

6. Haftverband gemäß einem der Ansprüche 1 bis 5, dadurch gekennzeichnet, daß das Trägermaterial aus einer Folie, einem Gewebe oder Vlies besteht.

7. Haftverband gemäß Anspruch 6, dadurch gekennzeichnet, daß das Trägermaterial aus einer Polyurethanfolie besteht.

8. Haftverband gemäß einem der Ansprüche 1 bis 7, dadurch gekennzeichnet, daß das Trägermaterial auf seiner nichtklebenden Seite mit einem wiederablösbaren Hilfsträger (4) abgedeckt ist, der zumindest an zwei gegenüberliegenden Seiten mit überstehenden Griffleisten versehen ist.

9. Haftverband gemäß einem der Ansprüche 1 bis 8, dadurch gekennzeichnet, daß auf der klebenden Seite eine Wundauflage (2) angeordnet ist, welche kleiner ist als die Klebefläche, so daß klebende Ränder freibleiben.

10. Haftverband gemäß Anspruch 9, dadurch gekennzeichnet, daß die Wundauflage sich teilweise oberhalb des Einschnitts bzw. der Aussparung befindet und sich teilweise noch entlang der Seitenränder des Einschnitts bzw. der Aussparung erstreckt.

11. Haftverband gemäß Anspruch 10, dadurch gekennzeichnet, daß die Wundauflage sich entlang etwa 2/3 - 3/4 der Länge des Einschnitts bzw. der Aussparung erstreckt.

12. Haftverband gemäß einem der Ansprüche 1 bis 11, dadurch gekennzeichnet, daß die Wundauflage und/oder die Selbstklebeschicht eine entzündungshemmende Substanz enthält.

13. Haftverband gemäß einem der Ansprüche 1 bis 12, dadurch gekennzeichnet, daß die Schutzabdeckung 3-teilig ausgebildet ist und die beiden Teilungslinien jeweils parallel zu dem Einschnitt bzw. der Aussparung verlaufen, jedoch versetzt zu diesen in der linken und rechten Hälfte des Verbandes.

## Claims

1. Adhesive dressing for intravenous catheters or the like comprising a carrier material (1) which has, on its under-side, a self-adhesive layer (5) provided with a protective covering (6) which is in at least two parts and can be peeled off, and has a central incision which starts from one edge of the dressing and partly separates this, characterized in that the dividing line (3) of the protective covering runs parallel to the incision, but displaced therefrom, in one half of the dressing.

2. Adhesive dressing according to Claim 1, characterized in that the incision is in the form of a longitudinal recess of any desired shape.

3. Adhesive dressing according to Claim 1 or 2, characterized in that the incision is in the form of a U-shaped recess.

4. Adhesive dressing for intravenous catheters or the like comprising a carrier material (1) which has, on its under-side, a self-adhesive layer (5) provided with a protective covering (6) which is in at least two parts and can be peeled off, characterized in that it has, on approximately the central line in one half of the dressing, a preferably longitudinal recess, and in that the dividing line (3) of the protective covering runs parallel to the recess, but displaced therefrom, in one half of the dressing.

5. Adhesive dressing according to one of Claims 1 to 4, characterized in that the protective covering parts are each provided with holding flaps (7) along their dividing line.

6. Adhesive dressing according to one of Claims 1 to 5, characterized in that the carrier material comprises a film, a woven fabric or a non-woven.

7. Adhesive dressing according to Claim 6, characterized in that the carrier material comprises a polyurethane film.

8. Adhesive dressing according to one of Claims 1 to 7, characterized in that the carrier material is covered on its non-adhesive side with a redetachable auxiliary carrier (4) which is provided with projecting gripping strips at least on two opposite sides.

9. Adhesive dressing according to one of Claims 1 to 8, characterized in that a wound covering (2) which is smaller than the adhesive area, so that adhesive edges remain free, is disposed on the adhesive side.

10. Adhesive dressing according to Claim 9, characterized in that the wound covering is partly above the incision or the recess and partly also extends along the side edges of the incision or of the recess.

11. Adhesive dressing according to Claim 10, characterized in that the wound covering extends along about 2/3 - 3/4 of the length of the incision or of the recess.

12. Adhesive dressing according to one of Claims 1 to 11, characterized in that the wound covering and/or the self-adhesive layer contains an anti-inflammatory substance.

13. Adhesive dressing according to one of Claims 1 to 12, characterized in that the protective covering is in the form of 3 parts and the two dividing lines each run parallel to the incision or the recess, but displaced therefrom, in the left and right half of the dressing.

## Revendications

1. Pansement adhésif pour cathéter intraveineux ou articles analogues fait d'une matière de substrat (1) présentant une couche autoadhésive (5) sur sa face inférieure, qui est pourvue d'un recouvrement protecteur enlevable (6), au moins en deux parties, et une coupure médiane partant d'un bord du pansement et le divisant en partie, caractérisé en ce que la ligne de séparation (3) du recouvrement protecteur s'étend dans une moitié du pansement, parallèlement à la coupure, mais décalée de celle-ci.

2. Pansement adhésif suivant la revendication 1, caractérisé en ce que la coupure a la forme d'une échancrure oblongue de configuration quelconque.

3. Pansement adhésif suivant la revendication 1 ou 2, caractérisé en ce que la coupure a la forme d'une échancrure en U.

4. Pansement adhésif pour cathéter intraveineux ou articles analogues fait d'une matière de substrat (1) présentant une couche autoadhésive (5) sur sa face inférieure, qui est pourvue d'un recouvrement protecteur enlevable (6), au moins en deux parties, caractérisé en ce qu'il présente, environ sur sa ligne médiane et dans une moitié de sa surface, une échancrure, de préférence oblongue, et que la ligne de séparation (3) du recouvrement protecteur s'étend dans une moitié du pansement, parallèlement à l'échancrure, mais décalée de celle-ci.

5. Pansement adhésif suivant l'une quelconque des revendications 1 à 4, caractérisé en ce que les parties du recouvrement protecteur sont chacune pourvue, le long de leur ligne de séparation, de languettes de prise (7).

6. Pansement adhésif suivant l'une quelconque des revendications 1 à 5, caractérisé en ce que la matière de substrat est faite d'une pellicule, d'un tissu ou d'un non-tissé.

7. Pansement adhésif suivant la revendication 6, caractérisé en ce que la matière de substrat est faite d'une pellicule de polyuréthanne.

8. Pansement adhésif suivant l'une quelconque des revendications 1 à 7, caractérisé en ce que la matière de substrat est recouverte, sur sa face non adhésive, d'un substrat auxiliaire détachable (4) qui est pourvu de bandes de préhension dépassantes sur au moins deux côtés opposés.

9. Pansement adhésif suivant l'une quelconque des revendications 1 à 8, caractérisé en ce que sur son côté adhésif est disposé un pansement proprement dit (2) qui est plus petit que la surface adhésive, de sorte que des bords adhésifs subsistent.

10. Pansement adhésif suivant la revendication 9, caractérisé en ce que le pansement proprement dit se trouve en partie au-dessus de la coupure ou de l'échancrure et s'étend en partie également le long des bords latéraux de la coupure ou l'échancrure.

11. Pansement adhésif suivant la revendication 10, caractérisé en ce que le pansement proprement dit s'étend sur environ 2/3 à 3/4 de la longueur de la coupure ou échancrure.

12. Pansement adhésif suivant l'une quelconque des revendications 1 à 11, caractérisé en ce que le pansement proprement dit et/ou la couche autoadhésive contiennent une substance anti-inflammatoire.

13. Pansement adhésif suivant l'une quelconque des revendications 1 à 12, caractérisé en ce que le recouvrement protecteur est en trois parties et les deux lignes de séparation s'étendent chacune parallèlement à la coupure ou échancrure, mais sont décalées par rapport à celle-ci dans la moitié gauche et la moitié droite du pansement.
